# EUROPEAN PATENT SPECIFICATION

(11) **EP 1 377 366 B1**
(45) Date of publication and mention of the grant of the patent: **21.03.2007**
(21) Application number: 02718287.2
(22) Date of filing: 02.04.2002
(51) Int. Cl.: B01J 7/02, B01J 3/00

(54) **SUPERCRITICAL FLUIDS OR DENSE PHASE GASES FORMED FROM LIQUID PRECURSORS**
SUPERKRITISCHE FLUIDE ODER DICHTE GASPHASEN AUS FLÜSSIGEN VORLÄUFERN
GAZ ET MELANGES DE GAZ POUVANT SPECIFIQUEMENT ETRE CONVERTIS EN FLUIDES SUPERCRITIQUES OU EN GAZ A PHASE QUASI-LIQUIDE FORMES A PARTIR DE PRECURSEURS LIQUIDES, APPAREIL PERMETTANT DE PRODUIRE CES GAZ ET CES MELANGES DE GAZ ET PRODUITS ELABORES A L'AIDE DE CES DERNIERS

(30) Priority: 31.03.2001 GB 0108151
(43) Date of publication of application: 07.01.2004
(73) Proprietor: The University of Nottingham, Nottingham NG7 2RD (GB)
(72) Inventor: POLIAKOFF, Martyn, Nottinghamshire NG9 1BU (GB); HYDE, Jason Robert, Sherwood, Nottingham NG5 3DU (GB)
(74) Representative: Lawrence, John
(86) International application number: PCT/GB2002/001246
(87) International publication number: WO 2002/078832

(56) References cited:
- WO-A-98/15509

## Description

This invention relates to the production of a gas or mixture of gases, typically but not necessarily for chemical reaction or chromatography, extraction or impregnation / modification of solid substrates; and to apparatus for making gas mixtures and or reacting gases in a gas mixture, and to materials made from the reactions.

The invention arose from considerations relating to the production of supercritical fluids and it is convenient to discus the invention in that context, but is not restricted to supercritical fluids.

It is sometimes desired to have a mixture of gases. This may be because it is desired to react the gases together, or with another substance. It may be because the gases are wanted for some other purpose. It is currently difficult to generate gas mixtures with a controlled composition and our invention is particularly useful in this respect.

Currently, supercritical fluid or dense phase gas formation is achieved by compressing a gas such as carbon dioxide (or a mixture of gases) to, near or above its critical pressure and heating to, near or above its critical temperature. This process requires the use of gas storage cylinders and equipment capable of withstanding high pressure. Also, the use of high-pressure pumps is required to achieve the supercritical state. Currently laboratory-scale supercritical fluids reactors are limited by the control of dosing of secondary gases into the supercritical fluid, and mass-flow control is difficult. An example of such a reactor can be found in WO 98/15509.

Supercritical fluids can be used as a medium for performing chemical reactions such as hydrogenation, acylation, alkylation, transesterification, etherification and cyclisation, or for use, for example in chromatography, extraction and impregnation. On a small scale, such as is currently used for laboratory screening, it is difficult both to meter the flow of gas and accurately to measure and maintain the composition of the fluid. We have unexpectedly found that at least some of the foregoing disadvantages can be eliminated or reduced by producing supercritical fluids from liquid precursors instead of from gases. This invention circumvents problems by use of liquid precursor(s) to generate the dense phase gas mixture or supercritical fluid.

According to a first aspect of the invention there is described the use of a supercritical fluid or dense phase fluid medium chemical reactor comprising: a reaction vessel adapted for performing chemical reactions in dense phase or supercritical fluids;
a gas generator comprising an inlet for liquid chemical precursor;
a gas communication channel from the gas generator to the reactor vessel; and
at least one liquid chemical precursor reservoir communicated to the inlet;
to produce a gas from chemical reaction or decomposition of one or more liquid chemical precursors in the gas generator vessel, and using the gas so produced in a supercritical fluid or dense phase fluid medium reaction.

According to another aspect of the invention there is provided a method of performing a supercritical or dense phase reaction comprising the steps of:
storing one or more liquid chemical precursor(s) to a gas in liquid form;
turning the liquid chemical precursor(s) into said gas, the gas being the product of a chemical reaction or decomposition of the liquid chemical precursor(s); and
using the gas produced in a supercritical or dense phase reaction,
wherein the liquid chemical precursor(s) is/are turned into the gas substantially at a time of demand for said gas.
The liquid precursor may be decomposed over a catalyst, or alternatively by means of energy input, for example, heat, light, sound, another energy source.

The gas or gas mixture produced may be conveyed to a reaction chamber or zone for the performance of chemical reactions. The gas or gas mixture may take part in the reactions or not.

We may also provide a supercritical fluid or dense phase gas made by the method described in the preceding paragraphs. We may also provide a reaction product of a chemical reaction that occurs in a supercritical fluid or dense phase gas produced by the method.

The liquid precursors are selected according to the chemical reaction and supercritical fluid desired, In principle, the precursor may be any substance that decomposes, can be decomposed or reacted to form a gas. For example, the liquid precursor may be an organic acid, or a salt or an ester of an organic acid, for example formic acid. For performing hydrogenation reactions the precursors are preferably formic acid and ethyl formate. Alternatively, methyl formate, ethyl formate, propyl formate or dimethyl carbonate, or any mixture thereof, may be used to yield gas mixtures of methane and carbon dioxide, ethane and carbon dioxide, carbon monoxide and propane and water, methane and carbon dioxide respectively.

When the decomposition method involves a catalyst, the catalyst may comprise any catalytically-active species. The catalyst may comprise rhodium, iridium, palladium or platinum. The catalyst is preferably supported, in a finely-divided condition, on an inert support. Such an inert support may be silica, or alumina, or an alumosilicate.

Formic acid decomposes over a metal catalyst at elevated temperature to form carbon dioxide and hydrogen. Under the conditions of the decomposition in accordance with one aspect of the present invention, a single-phase gas mixture is obtained. The gas mixture in this case comprises carbon dioxide and hydrogen in a 1:1 ratio. Variation of that ratio is impossible to achieve when the liquid precursor is formic acid alone and formation of a supercritical fluid is difficult. However, we have found that ethyl formate decomposes, under similar conditions, to form CO₂ and ethane (C₂H₆). Ethane has a similar critical temperature to CO₂.

Thus, when ethyl formate and formic acid are decomposed simultaneously, a gas mixture is formed (CO₂ + H₂ from the formic acid and CO₂ and C₂H₆ from the ethyl formate) which has more easily accessible critical conditions and is more easily formed into a supercritical fluid.

The stoichiometry of said supercritical fluid, i.e. the ratio of CO₂ and ethane to H₂, may be controlled by adjusting the rate at which the formic acid and the ethyl formate flow over the same catalyst bed.

It is important in this example that the precursors are not pre-mixed before they enter the reaction chamber, to prevent undesirable hydrolysis reactions.

This method may be used such that the reactions may be carried out, in dense phase gases or supercritical fluids for example, hydrogenation reactions, Friedel- Crafts acylation and alkylation reactions, transesterification, etherification, hydrogenolysis, deprotection and cyclisation reactions, depending upon the composition of the supercritical fluid formed by the chosen precursor(s) and on the catalyst employed.

By "at or near" to the gas mixing, or gas receiving, chamber we mean near enough to greatly reduce the volume of the components of the gas mixture present at a given time when compared with the situation where the components are held outside of the mixing chamber in storage cylinders or tanks. Preferably we react or decompose the precursor into gaseous form upon demand: - i.e. when it is desired to produce the gas. This avoids the need to store gas for a long time prior to using the gas.

The gas receiving chamber may be a zone or region of a pipe, or it may be an enlarged chamber, of greater cross-sectional area than that of a first and/or second precursor delivery conduit. Delivery conduits may deliver liquid components to the chamber where the liquid components gasify, or they may deliver gaseous first and/or second components to the chamber. There may be 2, 3, 4, 5, or more components.

The reaction may occur with the gas components in a gaseous or fluid state. Alternatively the gas mixture may be in a supercritical fluid, or dense phase gas, state.

Preferred embodiments of the invention will now be illustrated, merely by way of example, but should not be taken as limiting on the scope of this method to one skilled in the art, in the following description and with reference to the accompanying drawings, of which:-
**Figure 1** shows a flow diagram of a reactor for the production and subsequent use of supercritical fluids according to the present invention;
**Figures 2A to 2C** show modifications of feeds of precursor liquids;
**Figure 3** shows a modified plant similar to that of Figure 1;
**Figure 4** shows a computer-controlled micro-reactor plant; and
**Figure 5a - 5c** shows different gas generator and chemical reactor configurations.

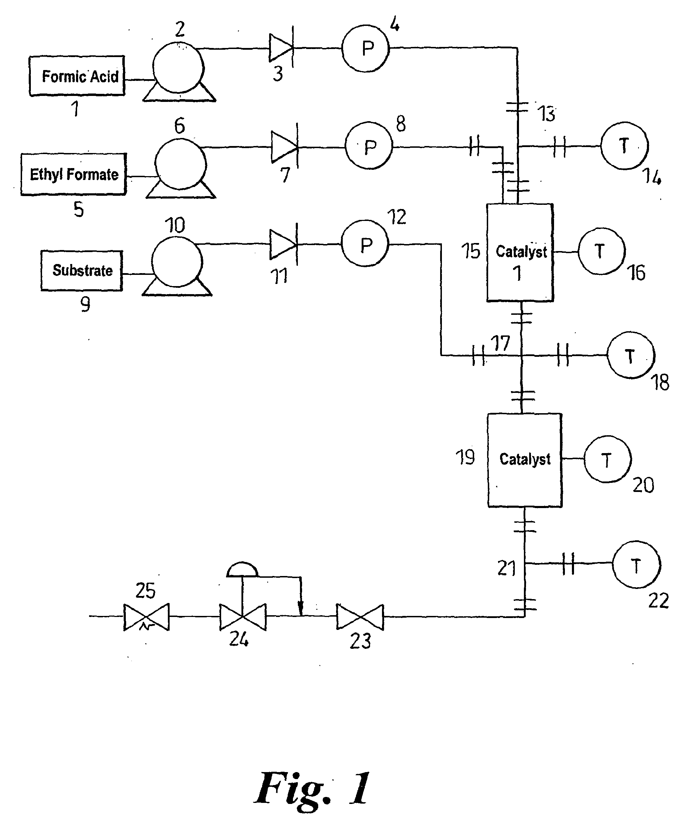

Referring to Figure 1, the reactor comprises two reservoirs, 1 and 5, for the liquid precursors and a reservoir 9, for a substrate. High-pressure pumps 2, 6 and 10 are each provided with a non-return valve 3, 7 and 11 respectively and a pressure monitor, 4, 8 and 12 respectively, in order to charge the reactor with the precursors and the substrate. Two heated catalytic chambers 15 and 19 are provided, each having a temperature monitoring apparatus 16 and 20 respectively.

Introduction of the liquid precursors prior to exposure to the catalyst is achieved in a first cruciform pipework intersection, shown schematically at 13. Mixing of the precursors before introduction to the catalyst bed is avoided by use of internal pipework. The temperature of the input of the precursors is monitored by temperature monitor 14. The inlet pipework of one precursor may direct the one precursor away from an introduced stream of a second precursor.

Mixing of the substrate with the products emerging from catalyst chamber 1 is achieved in a second cruciform pipework intersection, shown schematically at 17. The temperature of the substrate input is monitored by temperature monitor 18.

The product output from catalyst chamber enters a T-shaped pipework intersection 21, where its temperature is monitored by temperature monitor 22. The output product can be tapped off at sample collector 25.

A back pressure regulator 24 is fitted between the tap 23 and sample collector 25, in order to maintain a constant pressure within the reactor.

Chamber 15 is a gas-production chamber, and chamber 19 is a chemical reactor chamber in which the gases react with the substrate.

Of course, in other arrangements only one chamber may be necessary, but a plurality of chambers allows different conditions to exist in the different chambers. There may be more than one reaction chamber. The gas mixture produced may not take part in the reaction - for example it may be a supercritical fluid or dense phase gas solvent in which reactions of other chemicals occurs.

### Examples

Examples 1 and 2 below illustrate the use of supercritical fluids according to the present invention in the hydrogenation of trans-cinnamaldehyde and the effect of changing the flow rates of the precursors (formic acid and ethyl formate) on the yield of the desired product (hydrocinnamaldehyde) and on the amount of by-product (3-phenyl-1-propenol).

### Example 1: Hydrogenation of trans-cinnamaldehyde

The equipment was set up as shown in Figure 1. The catalyst chamber 1 (15) was charged with Deloxan AP II (Pt) 5% and maintained at a temperature of 400°C (16). Catalyst chamber 2 (19) was charged with Deloxan AP II (Pd) 5% and maintained at a temperature of 180°C (20). Formic acid and ethyl formate (precursor materials) were each charged to separate pumps (2 and 6) and entered catalyst chamber 1 (15) at flow rates of 0.2 and 0.2 ml/min respectively. The pressure of the reactor was maintained at 200 bar by means of back pressure regulator (24). At this temperature and pressure a supercritical liquid or dense phase fluid is formed (carbon dioxide, hydrogen, and ethane). Trans-cinnamaldehyde was charged to the substrate pump (10) from the reservoir. This was pumped into catalyst chamber 2 (19) at a flow rate of 0.05 ml/min. A solution containing the product was collected from the exit of the back pressure regulator (24) in an ice-cooled vial (25). Analysis of the solution by gas chromatography and ¹H NMR spectroscopy showed that the product was hydrocinnamaldehyde and that the yield was 65%. Gas chromatography and NMR analysis identified a yield of 30% of a second product, 3-phenyl-1-propenol.

### Example 2: Hydrogenation of trans-cinnamaldehyde

The equipment was set up as shown in Figure 1. The catalyst chamber 1 (15) was charged with Deloxan AP II (Pt) 5% and maintained at a temperature of 400°C (16). Catalyst chamber 2 (19) was charged with Deloxan AP II (Pd) 5% and maintained at a temperature of 180°C (20). Formic acid and ethyl formate were each charged to separate pumps (2 and 6) and entered catalyst chamber 1 (15) at flow rates of 0.1 and 0.4 ml/min respectively. The pressure of the reactor was maintained at 200 bar by means of a back pressure regulator (24). Trans-cinnamaldehyde was charged to the substrate pump (10). This was pumped into catalyst chamber 2 (19) at a flow rate of 0.05 ml/min. A solution containing the product was collected from the exit of the back pressure regulator (24) in an ice cooled vial (25). Analysis of the solution by gas chromatography and ¹H NMR spectroscopy showed that the product was hydrocinnamaldehyde and that the yield was 90%. Gas Chromatography and NMR analysis identified a yield of only 5% of a second product, 3-phenyl-1-propenol. Thus, changing the precursor flow-rates resulted in significant improvement in the yield of the desired product.

### Example 3: Production of cyclohexane without ethyl formate

The equipment was set up as in Figure 1. The catalyst chamber 1 (15) was charged with Deloxan AP II (Pt) 5% and maintained at a temperature of 400C (16). Catalyst chamber 2 (19) was charged with Deloxan AP II (Pd) 5% and maintained at a temperature of 80°C (20). Formic acid was charged to pump (2) and entered catalyst chamber 1 (15) at a flow rate of 0.2 ml/min. The pressure of the reactor was maintained at 80 bar by means of a back pressure regulator (24). Cyclohexene was charged to the substrate pump (10). This was pumped into catalyst chamber 2 (19) at a flow rate of 0.5 ml/min. A solution containing the product was collected from the exit of the back pressure regulator (24) in an ice cooled vial (25). Analysis of the solution by gas chromatography and ¹H NMR spectroscopy showed that the product was cyclohexane and that the yield was 100%.

### Example 4: Production of tetrahydrofuran without formic acid (etherification)

The equipment was set up as in Figure 1 and maintained. The catalyst chamber 1 (15) was charged with Deloxan AP II (Pt) 5% and maintained at a temperature of 400C (16). Catalyst chamber 2 (19) was charged with Amberlyst-15^{™} and maintained at a temperature of 180C (20). Ethyl formate was charged to pump (6) and entered catalyst chamber 1 (15) at a flow rate of 0.4 ml/min. The pressure of the reactor was maintained at 200 bar by means of a back pressure regulator (24). 1,4-butanediol was charged to the substrate pump (10). This was pumped into chamber 2 (19) at a flow rate of 0.5 ml/min. A solution containing the product was collected from the exit of the back pressure regulator (24) in an ice cooled vial (25). Analysis of the solution by gas chromatography and ¹HNMR spectroscopy showed that the product was tetrahydrofuran (THF) and that the yield was 100% (see Table 1).

### Example 5: Attempted Production of hydrocinnamaldehyde at 120 bar

The equipment was set up as in Figure 1. The catalyst chamber 1 (15) was charged with Deloxan AP II (Pt) 5% and maintained at a temperature of 400°C (16). Catalyst chamber 2 (19) was charged with Deloxan AP II (Pd) 5% and maintained at a temperature of 100°C (20). Formic acid was charged to pump (2) and entered catalyst chamber 1 (15) at a flow rate of 0.4 ml/min. The pressure of the reactor was maintained at 120 bar by means of a back pressure regulator (24). Trans-cinnamaldehyde was charged to the substrate pump (10). This was pumped into catalyst chamber 2 (19) at a flow rate of 0.05 ml/min. A solution was collected from the exit of the back pressure regulator (24) in an ice-cooled vial (25). Analysis of the solution by gas chromatography and NMR spectroscopy showed that no reaction had occurred.

### Example 6: Production of hydrocinnamaldehyde at 150 bar

The equipment was set up as in Figure 1. The catalyst chamber 1 (15) was charged with Deloxan AP II (Pt) 5% and maintained at a temperature of 400°C (16). Catalyst chamber 2 (19) was charged with Deloxan AP II (Pd) 5% and maintained at a temperature of 100°C (20). Formic acid was charged to pump (2) and allowed to enter catalyst chamber 1 (15) at flow rate of 0.4 ml/min. The pressure of the reactor was maintained at 150 bar by means of a back pressure regulator (24). Trans-cinnamaldehyde was charged to the substrate pump (10). This was pumped into catalyst chamber 2 (19) at a flow rate of 0.05 ml/min. A solution containing the product was collected from the exit of the back pressure regulator (24) in an ice-cooled vial (25). Analysis of the solution by gas chromatography and NMR spectroscopy showed that the product was hydrocinnamaldehyde and that the yield was 38%.

### Example 7: Production of 1,3,5, -trimethyl, 4-isopropylbenzene

The equipment was set up as in Figure 1. The catalyst chamber 1 (15) was charged with Deloxan AP II (Pt) 5% and held at a temperature of 400C (16). Catalyst chamber 2 (19) was charged with Nafion SAC-13 and held at a temperature of 200C (20). Ethyl Formate was charged to pump (2) and allowed to enter catalyst chamber 1 (15) at a flow rate of 0.4 mL/min. The pressure of the cell was maintained at 100 bar by means of a backpressure regulator (24). 1,3,5-trimethylbezene was charged to the substrate pump (10). This was pumped into chamber 2 (19) at a flow rate of 0.05 mL/min. A solution containing the product was collected from the exit of the backpressure regulator (24) in an ice cooled vial (25). Analysis of the solution by gas chromatography and ¹H NMR spectroscopy showed that a reaction had occurred; 1,3,5-trimethyl,4-isopropylbenzene was identified as the only product, with a conversion of 45%.

### Example 8: Production of 3 methyl,4-isopropylphenol

The equipment was set up as in Figure 1. The catalyst chamber 1 (15) was charged with Deloxan AP II (Pt) 5% and held at a temperature of 400C (16). Catalyst chamber 2 (19) was charged with Nafion SAC-13 and held at a temperature of 200C (20). Ethyl formate was charged to pump (2) and allowed to enter catalyst chamber 1 (15) at flow rate of 0.4 mL/min. The pressure of the cell was maintained at 300 bar by means of a back pressure regulator (24). M-cresol was charged to the substrate pump (10). This was pumped into chamber 2 (19) at a flow rate of 0.01 mL/min. A solution containing the product was collected from the exit of the back pressure regulator (24) in an ice cooled vial (25). Analysis of the solution by gas chromatography and ¹H NMR spectroscopy showed that the product was 3-methyl, 4-isopropyphenol as the only product and the yield was 30%.

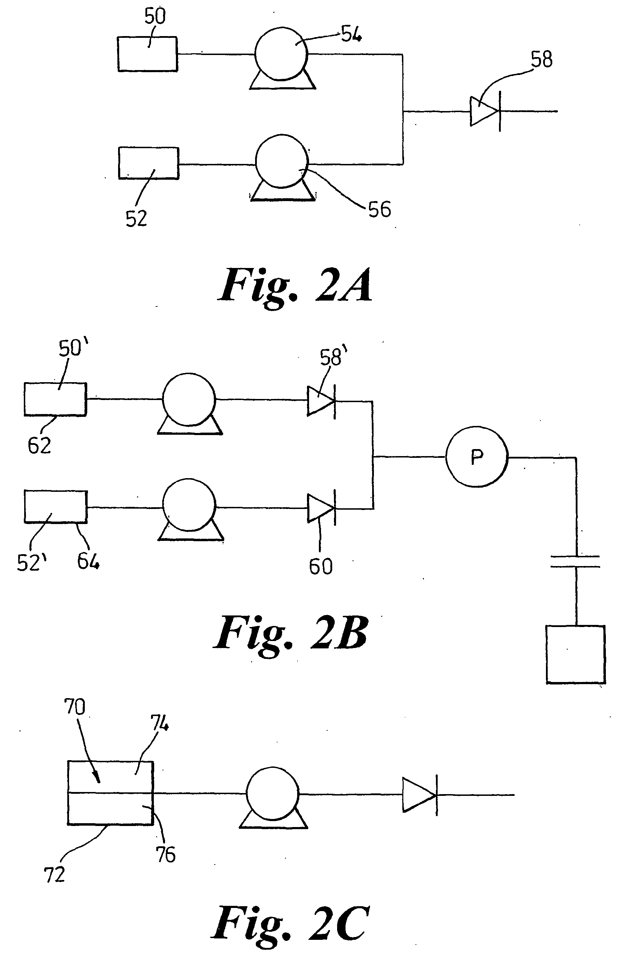

Figure 2A shows two precursor liquids 50 and 52 being metered and provided by precursor supply control meters 54 and 56 and shows the two liquids mixing before non-return valve 58. This may be permissible if the two liquids do not react together, or if they do react together but the reaction products are indeed what is wanted for introduction to the reaction chamber.

Figure 2B shows two liquids 50' and 52' mixing after passing through respective non-return valves 58' and 60. This prevents any contamination of the liquids 50' and 52' in liquid reservoirs 62 and 64.

Figure 2C schematically shows a precursor liquid 70 in a precursor liquid reservoir 72, where the liquid 70 is made of two different liquid substances, schematically shown as 74 and 76. The liquids 74 and 76 may be miscible or immiscible.

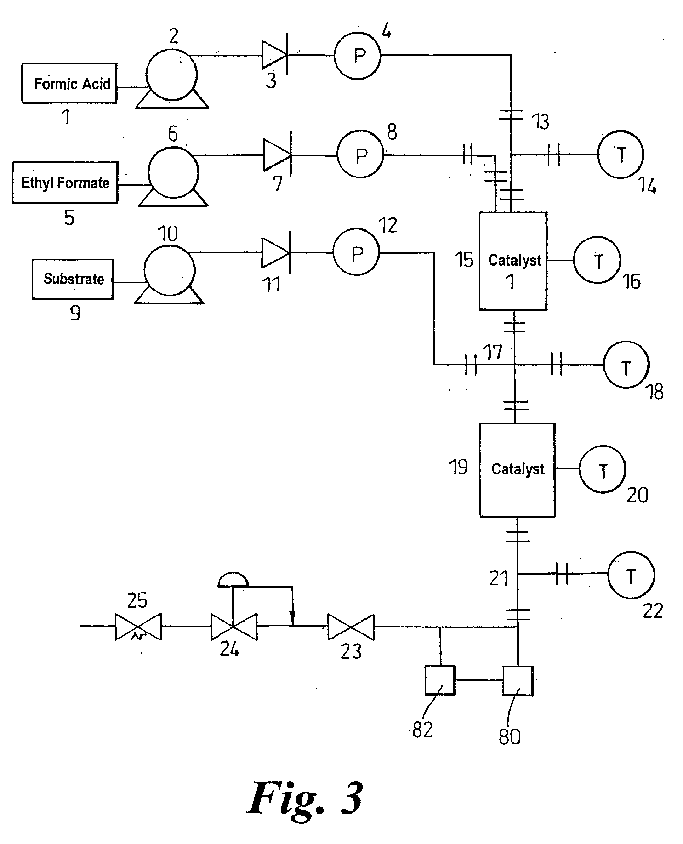

Figure 3 shows a micro-production facility similar to that of Figure 1, except that it has a gas liquid chromatograph (GLC) 80 which takes an on-line sample of what is being produced and analyses it. An infrared spectrometer 82 is also shown taking an on-line sample for on-line analysis.

The GLC and/or IR spectrometer could be at a different place in the production line, and/or samples from more than one place in the reaction sequences could be analysed.

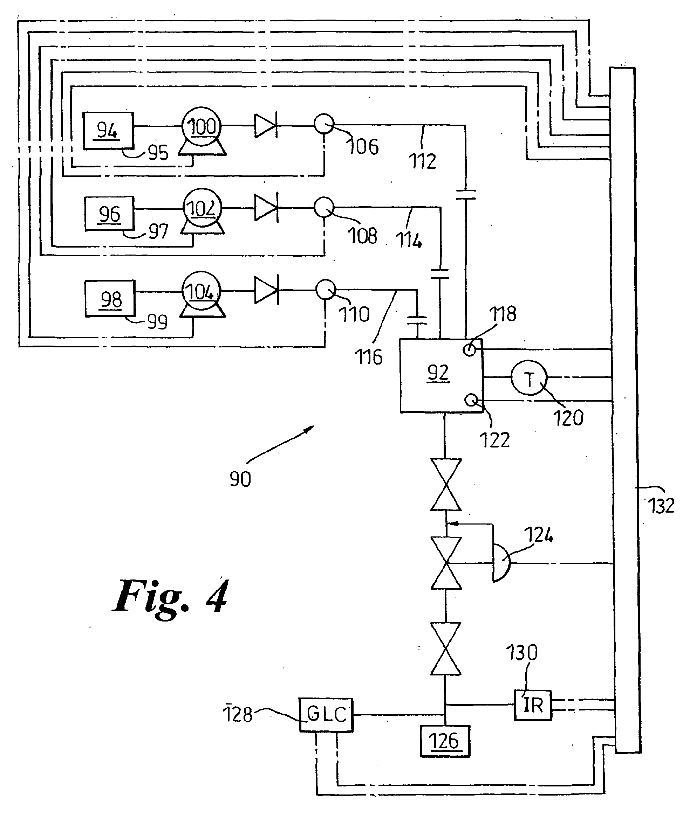

Figure 4 shows a micro-reactor plant 90 having a reaction chamber 92, precursor liquids 94, 96, 98 held in reservoirs 95, 97 and 99, metering pumps 100, 102, 104, pressure sensors 106, 108, 110, precursor inflow lines 112, 114, 116 leading to chamber 92, a pressure sensor 118 in chamber 92, a temperature controller 120 to control the temperature in chamber 92, a temperature sensor 122 in chamber 92, a back pressure regulator/pressure controller 124 controlling the pressure in chamber 92, a product collection reservoir 126, a gas/liquid chromatograph 128, an infrared spectrometer 130, and a microprocessor controller 132.

The controller 132 receives sensor signals from the sensors and output control signals to the pumps 100, 102, 104, the temperature controller 120, the pressure regulator 124, and the IR and GLC 128 and 130. The IR and GLC also provide information/analysis signals to the processor 132. The total volume of the chamber 92 and pipe work from reservoirs 92, 96, 98 to the chamber 92 is about 5ml.

The processor 132 can be programmed by a user to pump a known and carefully controlled amount of precursors 94, 96 and 98 (sometimes different amounts of each, sometimes the amounts may be the same) to the chamber 92 where the liquid precursors become gaseous. In many examples the pressure and temperature in chamber 92 is such that gaseous precursor materials, or their reaction products, become supercritical fluids. Chemical reaction occurs in the chamber 92, the course of the reactions occurring in chamber 92 being controlled by the temperature, pressure, and amounts of precursor allowed into the chamber. The reaction products are analysed in an on-line process, which may be continuous, by IR and/or GLC. The processor 132 can control conditions responsive to the feedback it receives from the sensors.

The processor 132 may be programmable by a user to produce different, selectable, reaction products, possibly from the same starting precursors, or possibly using different precursor substances.

By using small pilot, or laboratory, scale equipment for supercritical fluid reactions, but starting with relatively cheap or common starting materials (e.g. simple, small, organic molecules) has the advantage that if a reaction product of interest is made, it is relatively simple to produce the same reaction product from the same or similar starting precursors in a larger production facility. This approach contrasts with traditional laboratory synthesis of new substances, which typically uses complex and expensive starting materials to prove that a particular substance can be synthesised. Industrial chemists usually have to redesign the synthetic pathway typically using cheaper starting materials to make the large scale synthesis economically viable. In this new approach the laboratory synthesis chemist uses the same starting materials that are commonly available to the industrial chemist.

In some embodiments we may make the gases from liquid precursors, and mix them and/or react them with each other, and possibly with another substance, in a chamber.

This method may also be conveniently used to convert Paraformaldahyde (CHO)ₙ to CO + H₂, (an industrially important gas mixture known as Syngas). The ratio of CO to H₂ may be varied in Syngas for different purposes. Different customers want a different mix. In our method, the ratio of CO to H₂ can be easily varied by judicious use and combination of two or more of the following reactions;

(1) (CHO)ₙ → CO + H₂

(2) HCOOH → H₂O + CO.

(3) Indeed, any liquid → X + H₂

(4) or liquid → Y + CO

which can be used to vary the amount of H₂ versus CO in the resulting mixture of gases.
Thus, by varying the proportions of the liquid precursors (for example by changing their respective flow rates) we can vary the ratio of the resultant components within the gas mixture.

The reaction HCOOC₂H₅ → CO₂ + C₂H₆ is interesting because ethane has a relatively low critical point. We can use HCOOC₂H₅ specifically to create a supercritical ethane / carbon dioxide mixture. This mixture can be used as a vehicle for chemical reactions, or drying biological materials, to name but two uses.

Another useful reaction is: HCOOCH₃ → CH₄ + CO₂. We have made supercritical fluids using this. It will be appreciated that in the examples given in the Figures, the liquid precursors are delivered to the chamber 15 in liquid form and turn into gas in the chamber. They may then turn into a dense phase gas or supercritical fluid.

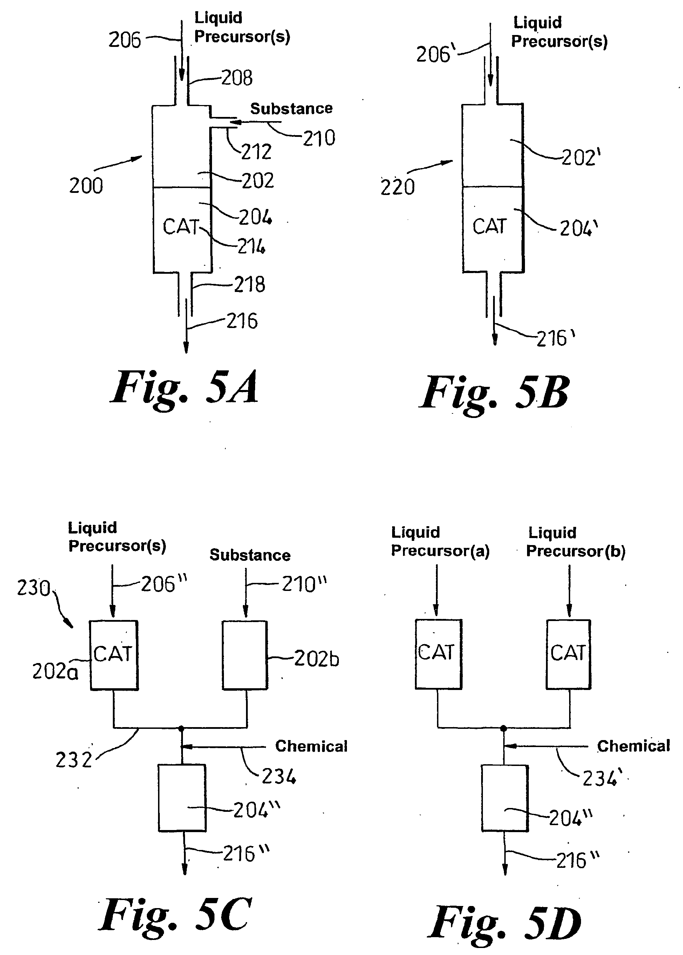

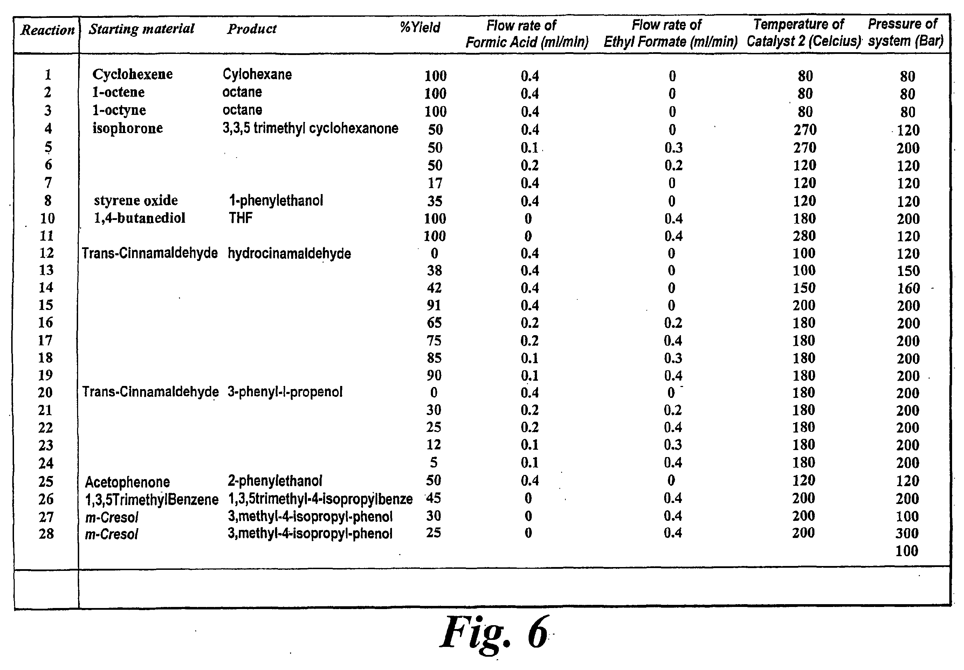

Figure 5A shows a chemical reactor 200 having a gas-production chamber 202 and a chemical reaction chamber 204. A liquid precursor 206 to a gas is introduced to the chamber 204 via port 208. A substance 210 is introduced to the chamber 202 via port 212. The gas produced from the precursor 206 and the substance 210 flow into the reaction chamber 204 where a chemical reaction takes place. The chamber 204 typically, but not always, has a catalyst 214 to facilitate the chemical reaction. A reaction product 216 leaves the chamber 204 via port 218.

The substance 210 may be a liquid precursor to a gas, or a gas itself, or a liquid, or a solid. The chemical reaction in chamber 204 preferably takes place in the supercritical fluid or dense phase gas state. The gas/substance mixture (or compound or mixture of compounds if they react) is preferably in the supercritical fluid or dense phase gas state in the chamber 202 and/or chamber 204. The liquid precursor 206 may be involved in the chemical reaction in the chamber 204, or it may not. The substance 210 may be involved in the chemical reaction or it may not (either of the gas from the precursor 206 or the substance may simply be a supercritical fluid /dense phase gas).

Figure 5B shows a reactor 220 that is similar to that of Figure 5A, except that no substance 210 is introduced. Similar components have been given similar reference numerals.

Figure 5C shows a variant on the chemical reactor arrangement. Reactor 230 has a first gas production chamber 202a and a second gas production chamber 202b. The chamber 202a receives liquid precursor 206". The chamber 202b receives a substance 210", which may be a gas, or a liquid precursor which experiences a chemical change to become a gas, or it may be a liquid which experiences a physical change to become a gas. Gas from chambers 202a and 202b mix in pipework 232 and a chemical substrate 234 is introduced at or prior to a chemical reactor 204".

Figure 5D shows a variant discussed above where the substance 210" of Figure 5C is itself a liquid precursor, precursor 206(b), and precursor 206" is also a liquid precursor (206a), both liquid precursors experiencing a chemical change to produce different gases or gas mixtures which are mixed prior to (or in some cases after) the introduction of a chemical substrate 234"'.

It will be appreciated that in preferred embodiments of the chemical reactors of Figures 5A to 5D the reactors comprise supercritical or dense phase fluid chemical reactors adapted to synthesise chemicals.

One way of looking at some embodiments of the invention is that we can provide supercritical fluids without gases as the starting point.

In summary, in one embodiment a gas or mixture of gases is produced by decomposition or chemical reaction of liquid precursor(s) which is converted to a dense phase or supercritical fluid state which can be used as a medium for chemical reactions. Changes in the gas composition may be made simply by modification of the metered flow rates or chemical composition of the liquid precursor(s).

The invention is performed, preferably, in a continuous flow reactor.

## Claims

1. Use of a supercritical fluid or dense phase fluid medium chemical reactor comprising: a reaction vessel adapted for performing chemical reactions in dense phase or supercritical fluids;
a gas generator comprising an inlet for liquid chemical precursor;
a gas communication channel from the gas generator to the reactor vessel; and
at least one liquid chemical precursor reservoir communicated to the inlet;
to produce a gas from chemical reaction or decomposition of one or more liquid chemical precursors in the gas generator vessel, and using the gas so produced in a supercritical fluid or dense phase fluid medium reaction.

2. Use of a reactor according to claim 1 wherein a mixture of gases is produced from chemical reaction or decomposition of one or more liquid precursors.

3. Use of a reactor according to claim 2 wherein the gas mixture is generated with a controlled composition.

4. Use of a reactor according to claim 1, claim 2, or claim 3 wherein an additional material introduction means is provided in the reactor vessel adapted to allow the introduction of an additional material.

5. Use of a reactor according to any preceding claim which comprises a hydrogenator.

6. Use of a reactor according to any preceding claim wherein a microprocessor or controller is provided adapted to control the temperature and pressure in the chamber or vessel, and to control the supply of the one or more liquid chemical precursor(s) to the gas generator.

7. Use of a reactor according to any preceding claim wherein pressure control means is provided, adapted to create a pressure in said chamber or vessel, and/or gas generator, which is greater than atmospheric pressure.

8. Use of a reactor according to claim 6 wherein said pressure control means comprises a back pressure regulator.

9. Use of a reactor according to any preceding claim wherein temperature control means is provided adapted to produce a temperature which is greater than 15 degrees Celsius.

10. Use of a reactor according to any preceding claim provided with a means of analysis adapted to analyse the composition of materials.

11. Use of a reactor according to any preceding claim wherein means is provided adapted to control the relative amounts of substance(s) introduced into the chamber.

12. Use of a reactor according to claim 6, or any claim dependent directly or indirectly from claim 6, wherein said microprocessor or controller is controllable by the user to cause the reactor to produce a gas mixture having a selected composition.

13. Use of a reactor according to claim 12, wherein there is more than one liquid precursor, and wherein the composition of the gas mixture produced is controlled by controlling the relative amounts of the liquid precursors introduced to the gas generator.

14. A method of performing a supercritical or dense phase reaction comprising the steps of:
storing one or more liquid chemical precursor(s) to a gas in liquid form;
turning the liquid chemical precursor(s) into said gas, the gas being the product of a chemical reaction or decomposition of the liquid chemical precursor(s); and
using the gas produced in a supercritical or dense phase reaction,
wherein the liquid chemical precursor(s) is/are turned into the gas substantially at a time of demand for said gas.

15. A method of performing a supercritical or dense phase reaction according to claim 14, wherein a mixture of gases having gas components is produced from the one or more liquid chemical precursor(s).

16. A method according to claim 15 wherein the composition of the generated gas mixture is controlled.

17. A method according to any one of claims 14 to 16 wherein the gas or mixture of gases is introduced into a chamber in which the pressure and temperature in the chamber are such that a dense phase gas or supercritical fluid is formed.

18. A method according to any one of claims 14 to 17, wherein the gas, or at least one of the gas components, is a decomposition product of a higher molecular weight liquid precursor molecule.

19. A method according to any one of claims 15 to 18 wherein there are a plurality of different liquid chemical precursors, each of which turns into a gaseous component of a mixture of gases.

20. The method of claim 19 wherein the composition of the gas mixture produced is controlled by controlling the relative amounts of liquid precursors.

21. The method of any one of claims 14 to 20 wherein the gas, or one of the gas components, produced is one or more of hydrogen, carbon dioxide, carbon monoxide, methane and ethane.

22. The method of any one of claims 14 to 21 wherein the supercritical or dense phase reaction carried out is any one of hydrogenation, Friedel-Crafts acylation or alkylation, transesterification, etherification, hydrogenolysis, deprotection and cyclisation.

23. A method according to any one of claims 14 to 22 wherein at least one liquid chemical precursor to the gas, or to a gas component, is from the group:-
organic acid, ester of an organic acid, formate, carbonate or salt of the afore mentioned.

24. A method according to any one of claims 14 to 23 wherein said liquid chemical precursor comprises one of, or at least one of;
(i) HCOOH
(ii) HCOOC₂H₅
(iii) HCOOCH₃
(iv) HCOOC₃H₇
(v) (CHO)ₙ, where n is greater than or equal to unity
(vi) H₂O₂

25. A method according to any one of claims 14 to 24 wherein said liquid chemical gas precursor comprises a mixture of formic acid and ethyl formate.

26. A method according to any one of claims 14 to 25 wherein a plurality of liquid chemical gas precursors are provided from the list:
(i) HCOOH
(ii) HCOOC₂H₅
(iii) HCOOCH₃
(iv) HCOOC₃H₇
(v) (CHO)ₙ, where n is greater than or equal to unity
(vi) H₂O₂

27. A method according to any one of claims 14 to 26 wherein said liquid chemical precursor liquid is decomposed or chemically reacted to form said gas or at least one said gas component.

28. A method according to any one of claims 14 to 27 wherein said liquid chemical precursor(s) react over a catalyst to form the said gas or said at least one gas component(s).

## Patentansprüche

1. Verwendung eines chemischen Reaktors für superkritische Fluid- oder Dichte-Phasen-Fluid-Medien, aufweisend
ein Reaktionsgefäß, geeignet zur Durchführung chemischer Reaktionen in Dichte-Phasen oder superkritischen Fluiden;
einen Gasgenerator umfassend einen Einlass für flüssigen chemischen Vorläufer, einen Gastransportkanal von dem Gaskanal zu dem Reaktionsgefäß; und
zumindest ein Vorratsgefäß für flüssigen chemischen Vorläufer, der mit dem Einlass in Verbindung steht,
um Gas durch eine chemische Reaktion oder Zerlegung eines oder mehrerer flüssiger chemischer Vorläufer in dem Gasgeneratorgefäß zu erzeugen, und Verwendung des auf diese Weise erzeugten Gases in einer superkritischen Fluid- oder Dichte-Phasen-Fluid-Reaktion.

2. Verwendung eines Reaktors nach Anspruch 1, wobei eine Mischung aus Gasen durch chemische Reaktion oder Zerlegung eines oder mehrerer flüssiger chemischer Vorläufer erzeugt wird.

3. Verwendung eines Reaktors nach Anspruch 2, wobei die Gasmischung mit einer gesteuerten Zusammensetzung erzeugt wird.

4. Verwendung eines Reaktors nach Anspruch 1, Anspruch 2 oder Anspruch 3, wobei ein zusätzliches Mittel zur Materialeinführung in dem Reaktionsgefäß bereit gestellt ist, das geeignet ist, die Einführung eines zusätzlichen Materials zu erlauben.

5. Verwendung eines Reaktors nach irgendeinem der vorstehende Ansprüche umfassend einen Hydrogenator.

6. Verwendung eines Reaktors nach irgendeinem der vorstehende Ansprüche, wobei ein Mikroprozessor oder eine Steuerung zur Verfügung steht, um die Temperatur und den Druck in der Kammer oder dem Gefäß zu steuern und um die Versorgung der einen oder mehreren flüssigen Vorläufer in dem Gasgenerator zu steuern.

7. Verwendung eines Reaktors nach irgendeinem der vorstehende Ansprüche, wobei ein Drucksteuerungsmittel zur Verfügung steht, das geeignet ist, um einen Druck in der Kammer oder dem Reaktionsgefäß und/oder dem Gasgenerator zu erzeugen, der größer ist als der Atmosphärendruck.

8. Verwendung eines Reaktors nach Anspruch 6, wobei das Drucksteuerungsmittel einen Gegendruckregler umfasst.

9. Verwendung eines Reaktors nach irgendeinem der vorstehenden Ansprüche, wobei ein Temperatursteuerungsmittel zur Verfügung steht, das geeignet ist, eine Temperatur zu erzeugen, die höher als 15 Grad Celsius ist.

10. Verwendung eines Reaktors nach irgendeinem der vorstehenden Ansprüche, ausgestattet mit einem Analysemittel, das geeignet ist, die Zusammensetzung von Materialien zu analysieren.

11. Verwendung eines Reaktors nach irgendeinem der vorstehende Ansprüche, wobei ein Mittel zur Steuerung der relativen Mengen an Substanz(en), die in die Kammer eingeführt wird/werden, zur Verfügung steht.

12. Verwendung eines Reaktors nach Anspruch 6 oder irgend einem Anspruch, der direkt oder indirekt von Anspruch 6 anhängig ist, wobei der Mikroprozessor oder die Steuerung durch den Anwender steuerbar ist, um den Reaktor zu veranlassen, eine Gasmischung mit einer ausgewählten Zusammensetzung zu erzeugen.

13. Verwendung eines Reaktors nach Anspruch 12, wobei es mehr als einen flüssigen Vorläufer gibt und wobei die Zusammensetzung der erzeugten Gasmischung gesteuert wird, indem die relativen Mengen des flüssigen Vorläufers gesteuert werden, die in den Gasgenerator eingeführt werden.

14. Verfahren zur Durchführung einer superkritischen oder Dichte-Phasen-Reaktion, umfassend die Schritte:
Bevorraten eines oder mehrerer flüssiger chemischer Vorläufer für ein Gas in flüssiger Form;
Umwandeln des flüssigen chemischen Vorläufers in das Gas, wobei das Gas das Produkt einer chemischen Reaktion oder einer Zerlegung des flüssigen chemischen Vorläufers ist und
Verwenden des erzeugten Gases in einer superkritischen oder Dichte-Phasen-Reaktion, wobei der/die flüssige(n) chemische(n) Vorläufer im Wesentlichen während einer Zeit des Bedarfs nach dem Gas in das Gas umgewandelt wird/umgewandelt werden.

15. Verfahren zur Durchführung einer superkritischen oder Dichte-Phasen-Reaktion nach Anspruch 14, wobei eine Mischung von Gasen mit Gaskomponenten aus dem einen oder mehreren flüssigen chemischen Vorläufer(n) erzeugt wird.

16. Verfahren nach Anspruch 15, wobei die Zusammensetzung der erzeugten Gasmischung gesteuert wird.

17. Verfahren nach irgendeinem der Ansprüche 14 bis 16, wobei das Gas oder die Mischung von Gasen in eine Kammer eingeführt wird, in der der Druck und die Temperatur in der Kammer derart sind, dass eine Dichte-Phase-Gas oder ein superkritisches Fluid gebildet wird.

18. Verfahren nach irgendeinem der Ansprüche 14 bis 17, wobei das Gas oder zumindest eine der Gaskomponenten ein Zerlegungsprodukt eines flüssigen Vorläufermoleküls von höherem Molekulargewicht ist.

19. Verfahren nach irgendeinem der Ansprüche 15 bis 18, wobei es eine Vielzahl unterschiedlicher flüssiger chemischer Vorläufer gibt, von denen sich jeder in eine gasförmige Komponente einer Gasmischung umwandelt.

20. Verfahren nach Anspruch 19, wobei die Zusammensetzung der erzeugten Gasmischung durch Steuern der relativen Mengen an flüssigen Vorläufern gesteuert wird.

21. Verfahren nach irgendeinem der Ansprüche 14 bis 20, wobei das erzeugte Gas oder eine der erzeugten Gaskomponenten eines oder mehrere aus Wasserstoff, Kohlendioxid, Kohlenmonoxid, Methan und Ethan ist.

22. Verfahren nach irgendeinem der Ansprüche 14 bis 21, wobei die ausgeführte superkritische oder Dichte-Phasen-Reaktion irgendeine aus Hydrogenierung, Friedel-Crafts-Acylierung oder -Alkylierung, Umesterung, Veretherung, Hydrogenolyse, Entschützung und Zyklisierung ist.

23. Verfahren nach irgendeinem der Ansprüche 14 bis 22, wobei zumindest ein flüssiger chemischer Vorläufer des Gases oder einer Gaskomponente aus der Gruppe umfassend:
- organische Säuren, Ester einer organischen Säure, Formiat, Karbonat oder Salz einer der vorgenannten Substanzen stammt.

24. Verfahren nach irgendeinem der Ansprüche 14 bis 23, wobei der flüssige chemische Vorläufer einen oder zumindest einen von
(i) HCOOH
(ii) HCOOC₂H₅
(iii) HCOOCH₃
(iv) HCOOC₃H₇
(v) (CHO)ₙ, wobei n größer oder gleich ist als eins
(vi) H₂O₂
umfasst.

25. Verfahren nach irgendeinem der Ansprüche 14 bis 24, wobei der flüssige chemische Gasvorläufer eine Mischung aus Ameisensäure und Ethylformiat umfasst.

26. Verfahren nach irgendeinem der Ansprüche 14 bis 25, wobei eine Vielzahl von flüssigen chemischen Gasvorläufern aus der Liste:
(i) HCOOH
(ii) HCOOC₂H₅
(iii) HCOOCH₃
(iv) HCOOC₃H₇
(v) (CHO)ₙ, wobei n größer oder gleich ist als eins
(vi) H₂O₂
bereitgestellt wird.

27. Verfahren nach irgendeinem der Ansprüche 14 bis 26, wobei die flüssige chemische Vorläuferflüssigkeit zerlegt oder chemisch abreagiert wird, um das Gas oder zumindest eine der Gaskomponenten zu bilden.

28. Verfahren nach irgendeinem der Ansprüche 14 bis 27, wobei der/die flüssige(n) chemische(n) Vorläufer über einem Katalysator reagieren, um das Gas oder die zumindest eine der Gaskomponente(n) zu bilden.

## Revendications

1. Utilisation d'un réacteur chimique à milieu fluide supercritique ou fluide en phase dense comprenant :
une cuve de réaction adaptée pour effectuer des réactions chimiques dans des fluides en phase dense ou supercritiques ;
un générateur de gaz comprenant un orifice d'entrée pour un précurseur chimique liquide ;
un canal de communication de gaz allant du générateur de gaz à la cuve de réacteur ; et
au moins un réservoir de précurseurs chimiques liquides en communication vers l'orifice d'entrée ;
pour produire un gaz à partir d'une réaction ou décomposition chimique d'un ou plusieurs précurseur(s) chimique(s) liquide(s) dans la cuve de générateur de gaz, et en utilisant le gaz ainsi produit dans une réaction en milieu fluide supercritique ou fluide en phase dense.

2. Utilisation d'un réacteur selon la revendication 1, dans laquelle un mélange de gaz est produit à partir d'une réaction ou décomposition chimiques d'un ou plusieurs précurseur(s) liquide(s).

3. Utilisation d'un réacteur selon la revendication 2, dans laquelle le mélange de gaz est généré avec une composition régulée.

4. Utilisation d'un réacteur selon la revendication 1, la revendication 2 ou la revendication 3, dans laquelle des moyens d'introduction de matériaux additionnels sont disposés dans la cuve de réacteur adaptée pour permettre l'introduction d'un matériau additionnel.

5. Utilisation d'un réacteur selon l'une quelconque des revendications précédentes, qui comprend un hydrogénateur.

6. Utilisation d'un réacteur selon l'une quelconque des revendications précédentes, dans laquelle un microprocesseur ou une unité de commande est prévu(e) adapté(e) pour commander la température et la pression dans la chambre ou cuve, et pour commander l'alimentation d'un ou plusieurs précurseur(s) chimique(s) liquide(s) dans le générateur de gaz.

7. Utilisation d'un réacteur selon l'une quelconque des revendications précédentes, dans laquelle des moyens de commande de pression sont prévus, adaptés pour créer une pression dans ladite chambre ou cuve, et/ou le générateur de gaz, qui est plus grande que la pression atmosphérique.

8. Utilisation d'un réacteur selon la revendication 6, dans laquelle lesdits moyens de commande de pression comprennent un régulateur de contre-pression.

9. Utilisation d'un réacteur selon l'une quelconque des revendications précédentes, dans laquelle des moyens de commande de température sont prévus, adaptés pour produire une température qui est plus grande que 15 degrés Celsius.

10. Utilisation d'un réacteur selon l'une quelconque des revendications précédentes, dotée de moyens d'analyses adaptés pour analyser la composition de matériaux.

11. Utilisation d'un réacteur selon l'une quelconque des revendications précédentes, dans laquelle des moyens sont prévus, adaptés pour commander les quantités relatives de substance(s) introduite(s) dans la chambre.

12. Utilisation d'un réacteur selon la revendication 6, ou toute revendication dépendant directement ou indirectement de la revendication 6, dans laquelle ledit microprocesseur ou ladite unité de commande peut être commandé(e) par l'utilisateur pour amener le réacteur à produire un mélange de gaz ayant une composition choisie.

13. Utilisation d'un réacteur selon la revendication 12, dans laquelle il existe plus d'un précurseur liquide, et dans laquelle la composition du mélange de gaz produit est commandée en commandant les quantités relatives des précurseurs liquides introduits dans le générateur de gaz.

14. Procédé de réalisation d'une réaction en phase supercritique ou dense comprenant les étapes consistant à :
stocker un ou plusieurs précurseur(s) chimique(s) liquide(s) d'un gaz sous forme liquide ;
transformer le(s) précurseur(s) chimique(s) liquide(s) en ledit gaz, le gaz étant le produit d'une réaction ou décomposition chimique du(des) précurseur(s) chimique(s) liquide(s) ; et
utiliser le gaz produit dans une réaction en phase supercritique ou dense,
dans lequel le(s) précurseur(s) chimique(s) liquide(s) est (sont) transformé(s) en le gaz sensiblement au moment de la demande en ledit gaz.

15. Procédé de réalisation d'une réaction en phase supercritique ou dense selon la revendication 14, dans lequel un mélange de gaz ayant des composants gazeux est produit à partir d'un ou plusieurs précurseur(s) chimique(s) liquide(s).

16. Procédé selon la revendication 15, dans lequel la composition du mélange de gaz généré est commandée.

17. Procédé selon l'une quelconque des revendications 14 à 16, dans lequel le gaz ou mélange de gaz est introduit dans une chambre dans laquelle la pression et la température dans la chambre sont telles qu'un gaz en phase dense ou un fluide supercritique est formé.

18. Procédé selon l'une quelconque des revendications 14 à 17, dans lequel le gaz, ou au moins l'un des composants gazeux, est un produit de décomposition d'une molécule de précurseur liquide à haute masse moléculaire.

19. Procédé selon l'une quelconque des revendications 15 à 18, dans lequel il existe une pluralité de précurseurs chimiques liquides différents, dont chacun se transforme en un composant gazeux d'un mélange de gaz.

20. Procédé selon la revendication 19, dans lequel la composition du mélange gazeux produit est commandée en commandant les quantités relatives de précurseurs liquides.

21. Procédé selon l'une quelconque des revendications 14 à 20, dans lequel le gaz, ou l'un des composants gazeux, produit est un ou plusieurs parmi l'hydrogène, le dioxyde de carbone, le monoxyde de carbone, le méthane et l'éthane.

22. Procédé selon l'une quelconque des revendications 14 à 21, dans lequel la réaction en phase supercritique ou dense réalisée est l'une quelconque parmi une hydrogénation, une acylation ou alkylation de Friedel-Crafts, une transestérification, une éthérification, une hydrogénolyse, une déprotection et une cyclisation.

23. Procédé selon l'une quelconque des revendications 14 à 22, dans lequel au moins un précurseur chimique liquide du gaz, ou d'un composant gazeux, est parmi le groupe :
d'un acide organique, d'un ester d'un acide organique, d'un formiate, d'un carbonate ou d'un sel des éléments mentionnés ci-avant.

24. Procédé selon l'une quelconque des revendications 14 à 23, dans lequel ledit précurseur chimique liquide comprend l'un parmi, ou au moins l'un parmi :
(i) HCOOH
(ii) HCOOC₂H₅
(iii) HCOOCH₃
(iv) HCOOC₃H₇
(v) (CHO)ₙ, où n est supérieur ou égal à l'unité
(vi) H₂O₂

25. Procédé selon l'une quelconque des revendications 14 à 24, dans lequel ledit précurseur chimique liquide de gaz comprend un mélange d'acide formique et de formiate d'éthyle.

26. Procédé selon l'une quelconque des revendications 14 à 25, dans lequel une pluralité de précurseurs chimiques liquides de gaz sont prévus à partir de la liste :
(i) HCOOH
(ii) HCOOC₂H₅
(iii) HCOOCH₃
(iv) HCOOC₃H₇
(v) (CHO)ₙ, où n est supérieur ou égal à l'unité
(vi) H₂O₂

27. Procédé selon l'une quelconque des revendications 14 à 26, dans lequel ledit liquide précurseur chimique liquide est décomposé ou chimiquement mis à réagir pour former ledit gaz ou au moins un dudit composant gazeux.

28. Procédé selon l'une quelconque des revendications 14 à 27, dans lequel ledit (lesdits) précurseur(s) chimique(s) liquide(s) réagit(réagissent) sur un catalyseur pour former ledit gaz ou ledit au moins un composant gazeux.
